(19) Europäisches Patentamt

European Patent Office

Office européen des brevets

(11) **EP 1 300 153 A1**

(12) **EUROPEAN PATENT APPLICATION**
published in accordance with Art. 158(3) EPC

(43) Date of publication:
09.04.2003 Bulletin 2003/15

(51) Int Cl.7: **A61K 31/727**

(21) Application number: 01969177.3

(86) International application number:
**PCT/CN01/00922**

(22) Date of filing: 08.06.2001

(87) International publication number:
**WO 01/093876 (13.12.2001 Gazette 2001/50)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **09.06.2000 CN 116404X**

(71) Applicant: **Shanghai Institute of Cell Biology, Cas Shanghai 200031 (CN)**

(72) Inventors:
• **GENG, Jianguo**
  **Shanghai 200031 (CN)**
• **WANG, Jianguo**
  **Shanghai 200031 (CN)**

(74) Representative: **Schrell, Andreas, Dr.**
  **Gleiss & Grosse**
  **Leitzstrasse 45**
  **70469 Stuttgart (DE)**

(54) **USE OF N-DESULFATED HEPARIN FOR TREATING OR PREVENTING INFLAMMATIONS**

(57)    The invention relates to the use of N-desulfated heparin for treating or preventing inflammation, which is based on the experimental results in animal acute abdominal inflammation model and animal bleed model. The N-desulfated heparin's anti-inflammation activity is better than or equal to that of the low molecular weight heparin, and has low activity of anti-coagulant. From a series of N-desulfated heparin of different N-sulfur-containing, a sample of the best anti-inflammation activity and the lowest anti-coagulant activity was selected. The invention solved the problem of bleeding in the use of heparin for treating of inflammation, and provided a new pathway to use heparin to prevent and treat inflammation.

EP 1 300 153 A1

Printed by Jouve, 75001 PARIS (FR)

## Description

The present invention relates to the pharmaceutical use, especially using N-desulfated heparin, which has the significantly reduced anticoagulant activity while preserving the anti-inflammation activity, for prevention and treatment of inflammation. The invention further relates to methods and means for testing of heparin and heparin derivatives with significantly reduced anticoagulant activities for therapeutic purposes.

DESCRIPTION OF BACKGROUND AND RELATED ART

Heparin is a highly sulfated natural polysaccharide that is first described by McLean in 1916. It has been used clinically as an anticoagulant for more than 50 years [McLean, Circulation 19, 75-78 (1959)]. It is synthesized in the endoplasmic reticulum as proteoglycans attached to the protein serglycine [Toledo and Dietrich, Biochim. Biophys, Acta 498, 114-122 (1977)]. Heparin is an intracellular product found in the secretary granules of mast cells often in complexation with basic proteins. In response to certain external signals, it is released exclusively from mast cells of the lung, intestine and liver during their degranulation. The released soluble heparin carries negative charges and is know to bind to more than one hundred proteins, including many growth factors and chemokines. Heparin can be isolated, in a relative economic way, from the highly vasculaturized tissues, such as porcine or bovine intestinal mucosa, or less commonly, from bovine lung tissues [Conrad, Heparin-Binding Proteins, Academic Press, San Diego (1998)].

Heparin is a mixture of polydisperse, unbranched polymers made up of repeating units of alternating disaccharides containing hexuronate (either $\beta$-D-glucuronic acid or $\alpha$-L-iduronic acid) and $\alpha$-D-glucosamine (either N-sulfated or N-acetylated). They are joined by (1, 4) glycosidic linkages. The major sites where sulfation may be present are at the 2-O position of the iduronic acid residues as well as the 2-N position and 6-O position of the glucosamine residues. In addition, it is occasionally sulfated on either the 2-O position of glucuronic acid residues or the 3-O position of the disulfated glucosamine residues [Tyrrell et al., Adv. Pharmacol, 46, 151-208 (1999)]. The linear glycosaminoglycan chains are covalently bound to serine residues of core proteins. The heparin polymer may contain 20-100 monosaccharide units per polysaccharide chain, and due to the long repeats of (ser-gly)$_n$ in the protein sequence, a high density of heparin chains can be attached to the serglycine core [Ruoslahti, J. Biol. Chem. 264, 13369-13372 (1989)]. However, the number and position of GAG chains vary with the protein core.

Besides heparin, there has another sulfated polysaccharide, called heparan sulfate, which is structurally similar to heparin and contains all of the structural motifs found in heparin. In contrast to heparin, it is normally secreted from cells. It is distributed ubiquitously on the surface of most animal cells and as a major component of the basement membranes in extracellular matrix. Heparin and heparan sulfate are members of a family of polysaccharides termed "glycosaminoglycans," or GAGs [Höök et al., Annu. Rev. Biochem. 53, 847-869 (1984)]. In addition to heparin and heparan sulfate, this family includes chondroitin-sulfate, dermatan sulfate and hyaluronic acid.

Heparin and heparan sulfate have the most heterogeneous structures in the GAG family. Heparan sulfate typically has a low level of N- and O-sulfation and retains more of the original N-acetylglucosamine and glucuronate residues. Compared to heparin, heparan sulfate generally has fewer and shorter GAG chains, which can be attached to a variety of core proteins, often in conjunction to chondroitin sulfate chains [Kjellén and Lindahl, Ann. Rev. Biochem. 60, 443-475 (1991)]. It can also be attached directly to the cell surface (via either a transmembrane domain of the core protein or a phosphatidyl-inositol likage) or they may be bound by specific receptors at the cell surface [Gallagher, Curr. Opin. Cell Biol. 1, 1201-1218 (1989)]. It has reported that different cells can have distinct subtypes of heparan sulfate chain, suggesting a possibility that they may play roles in mediating cellular interactions [Kim et al., Mol. Biol. Cell 5, 797-805 (1994); Engelmann et al., Biochim. Biophys. Acta Mol. Cell Res. 1267, 6-14 (1995); Archer et al., J. Anat. 189, 23-35 (1996)].

Compared to heparan sulfate, heparin is more highly sulfated and contains a greater percentage of iduronate residues that have been epimerized from glucuronate. Due to the differences in composition and the extent of sulfation, heparin is more highly charged than heparan sulfate. The higher percentage of iduronate residues in heparin also increases the relative flexibility of the polymer. This flexibility coupled with an increase in the presence of additional electrostatic interactions is presumed to provide a greater biological activity associated with iduronate-containing GAGs as compared to glucuronate containing counterparts [Casu et al., Trends Biochem. Sci. 13, 221-225 (1988)].

The most widely accepted and commercially exploited functions of heparin are as an anticoagulant. This action of heparin resides in its ability to regulate the activity of an endogenous coagulation cofactor, called antithrombin-III (AT-III), which inhibits many serine proteases involved in the coagulation cascade [Bourin and Lindahl, Biochem. J. 289, 313-330 (1993)]. Heparin can interact with AT-III, via a specific high-affinity pentasaccharide sequence that represents only a minor portion of any heparin chain, to form a complex that inhibits thrombin and Factor Xa much more effectively than AT-III alone. Heparin can also interact with another serine protease inhibitor, heparin cofactor II (HC-II), to further potentate the inhibition of thrombin.

Beyond its well-recognized anticoagulant activity, heparin and heparan sulfate have various non-anticoagulant actions. Among them are anti-inflammatory roles, including prevention of leukocyte adhesion [Tangelder and Arfors, Blood 77, 1565-1571 (1991); Ley *et al.*, Am. J. Physiol. 260, H1667-H1673 (1991); Arfors and Ley, J. Lab. Clin. Med., 121, 201-202 (1993); Teixeira and Hellewell, Br. J. Pharmacol. 110, 1496-1500 (1993); Nelson *et al.*, Blood. 82, 3253-3258 (1993); Seeds *et al.*, L Lipid Mediators 7, 269-278 (1995); Kitamura *et al.*, Eur. Sung. Res. 28, 428-435 (1996); Weber *et al.*, J. Cereb. Blood Flow Metab. 17, 1221-1229 (1997); Giuffrè *et al.*, J. Cell Biol. 136, 945-956 (1993)] and activation [Pasini *et al.*, Thromb. Res. 35, 527-537 (1984); Bazzoni *et al.*, J. Lab. Clin. Med. 121, 268-275 (1993); Riesenberg *et al.*, Br. Heart J. 73, 14-19 (1995); Ahmed *et al.*, *Am.* J. Respir. Crit Care Med. 155, 1848-1855 (1997)], inhibition of complement activation [Weiler *et al.*, J. Immunol. 148, 3210-3215 (1992); Teixeira *et al.*, J. Leukocyte Biol. 59, 389-396 (1996)], the maintenance of endothelial wall competence and integrity and the protection of vascular endothelial cells from a number of damaging substances, such as chemokines, histamine, bradykinin, bacterial endotoxin, lysosomal cationic proteins and oxygen free radicals [Engelberg, Semin. Thromb. Hernost. 11, 48-55 (1985); Hiebert and Liu, Semin. Thromb. Hemost. 17(suppl 1), 42-46 (1991); Tanaka *et al.,* Nature 361, 79-82 (1993); Webb *et al.*, Proc. Natl. Acad. Sci. USA 90, 7158-7160 (1993)].

For example, heparin and heparan sulfate are known to bind to cell adhesion molecules P-selectin, L-selectin and Mac-1 (CD11b/CD18) and to inhibit leukocyte adhesion mediated by these molecules [Skinner *et al.*, Biochem. Biophys. Res. Commun. 164, 1373-1379 (1989); Skinner *et al.*, J. Biol. Chem. 266, 5371-5374 (1991); Nelson *et al.*, Blood. 82, 3253-3258 (1993); Norgard-Sumnicht *et al.*, Science. 261, 480-483 (1993); Diamond *et al.*, J. Cell Biol. 130, 1473-1482 (1995); Giuffre *et al.*, J. Cell Biol. 136, 945-956 (1997); Koenig et al., J. Clin. Invest. 101, 877-889 (1998)]. Heparan sulfate, synthesized by endothelial cells [Castillo *et al.*, Biochem. J. 247, 687-693 (1987); Kinsella and Wight, Biochemistry 27, 2136-2144 (1988)], can bind to various chemokines and therefore optimally localize these chemotactic cytokines [Tanaka *et al.,* Nature 254, 79-82 (1993); Furie and Randolph, Am. J. Pathol. 146, 1287-1301 (1995)]. Heparin and heparin sulfate can bind to many biochemical constituents on endothelial cells and thus restore the negative charge of the endothelial cell surface made positive following tissue injury [Engelberg, Semin. Thromb. Hemost. 11, 48-55 (1985); Hiebert and Liu, Semin. Thromb. Hemost, 17(suppl 1), 42-46 (1991)].

In literature, heparin can by modified chemically in many ways. These modified heparin derivatives have been proved to be invaluable for the structural and functional studies. They include periodate oxidation, *N*-desulfation, *N*-deacetylation, modifications of *N*-unsubstituted heparinoids, 2-*O*-desulfation, 6-*O*-desulfation, carboxyl reduction and derivatization, epimerization, sulfate migration, and oversulfation [Conrad, Heparin-Binding Proteins, Academic Press, San Diego (1998)].

Tiozzo and his colleagues have reported the effect of heparin desulfation on the anticoagulant activities. N-desulfated heparin derivatives, compared with heparin as the starting material, have significantly reduced anticoagulant activities, but the anticoagulant activities of heparin and its derivatives have no absolute linear relationships with the N-sulfate contents for heparin chains. [Tiozzo *et al.*, Thromb. Res. 70, 99-106 (1993)].

Using chemical modifications, it has been shown that the non-anticoagulant heparin derivatives could inhibit rat arterial smooth muscle cell proliferation in vivo [Guyton *et al.*, Circ. Res. 46, 625-634 (1980)]. The 2-O-desulfated and 3-O-desulfated heparin derivatives had reduced anticoagulant activities, but preserved their heparanase-inhibitory, angiostatic, anti-tumor and anti-metastatic properties. (Masayuki *et al.*, U.S. Patent 5,795, 875 (1997); Lapierre *et al.*, Glycobiology 6, 355-366 (1996)].

In addition, Ahmed and his colleagues have reported that they could generate non-anticoagulant heparin by collection of the low affinity fraction of the intact, non-chemical modified heparin from the affinity resins conjugated with antithrombin III. The non-anticoagulant heparin thus prepared could inhibit antigen-induced acute bronchconstriction, airway hyperresponsiveness, and mass cell degranulation [Ahmed *et al.*, Am. J. Respir. Crit. Care Med. 155, 848-1855 (1997)].

Heparin and low molecular weight heparin can be used to treat inflammatory diseases, but have dangerous side effects of bleeding or hemorrhage in the tissues due to their potent anticoagulant activities, which limit their extensively clinical use for patients with inflammatory disorders. Many chemical modification methods were patented to reduce their_anticoagulant activities while preserving other biological properties. An example of O-desulfated heparin derivatives is described in U.S. Pat. No.5,795,875. These O-desulfated heparin derivatives, were shown to be useful for treating various diseases including inflammation. They had substantially unfragmented 6-O desulfated heparin, or 6-O desulfated heparin fragments, but still retained 5-30% anticoagulant activities of the heparin. Another example of O-desulfated heparin derivatives, preferably 2-O, 3-O desulfated heparin, were described by Holme et al. U.S. Pat. 5,296,471. They retained 5.5-23% anticoagulant activities when compared to heparin. However, these O-desulfated heparin derivatives still had quite potent anticoagulant activities.

It has been known that N-desulfated heparin had a significant reduced anticoagulant activity. However, the effects of removing N-sulfate on the anti-inflammation activity of heparin remain largely undetermined so far. Thus, the anti-inflammation activities of N-desulfated heparin derivatives, particularly in prevention and treatment of the inflammation diseases, deserve further investigations.

PURPOSE OF THIS INVENTION

The purpose of this invention is to provide a pharmaceutical use of N-desulfated heparin in prevention and treatment of inflammation. The anti-inflammation activity of N-desulfted heparin is better than or equal to low molecular weight heparin (LMH). However, it has a significant reduced anticoagulant activity. The present invention thus provides a good possibility of using N-desulfated heparin for prevention and treatment of inflammation.

Furthermore, the present invention, Prevention and Treatment of Inflammation by N-desulfated Heparin, is based on the chemical modification method to remove the N-sulfate of heparin chain. Here, eight heparin derivatives were screened for the most potent anti-inflammation activity and for the lowest anticoagulant activity using the acute peritonitis mouse model and the bleeding time assay. The results indicated the effects of desulfation on their anti-inflammation activities and their anticoagulant activities. N-desulfated heparin apparently has more potent anti-inflammation activity than LMH. At the same time, it has a lower anti-anticoagulant activity.

Heparin as the starting material was chemically modified to remove N-sulfate [Nagasawa et al., <u>Carbohydr. Res. 36,</u> 265-271,1976; Inoue and Nagasawa, <u>Carbohydr. Res. 46</u>, 87-95(1976)]. The anticoagulant activities of heparin, LMH and all heparin derivatives were determined. Table 1 showed the results of the absolute and the relative contents of N-sulfate as well as the aPTT times.

Table 1:

| Heparin and its derivatives | Absolute N-sulfate content (%) | Relative N-sulfate content (%) | 2aPTT (µ g/ml) |
|---|---|---|---|
| UFH | 3.25 | 100 | 0.56 |
| LMH | 3.54 | 109.1 | 3.3 |
| ① | 0.62 | 19.0 | 20.5 |
| ② | 0.55 | 17.0 | 34.8 |
| ③ | 1.50 | 46.2 | 9.0 |
| ④ | 0.36 | 11.0 | 115 |
| ⑤ | 1.99 | 61.2 | 2.3 |
| 6 | 2.31 | 71.0 | 6.5 |
| 7 | 2.80 | 86.2 | 1.7 |
| 8 | 2.08 | 64.0 | 6.4 |
| UFH: unfractionated heparin; LMH: low molecular weight heparin APTT: activated partial thromboplastin time | | | |

As shown in Table 1, all N-desulfated heparin derivatives had significant reduced anticoagulant activities when they were compared with heparin as the starting material. The relative N-sulfate contents of No. 3, 5, 6, 7, 8 samples were 46.2, 61.2, 71.0, 86.2 and 64.0%, respectively; their anticoagulant activities were 1/16, 1/4, 1/12, 1/3 and 1/11 of heparin, individually. The relative N-sulfate contents of No.1, 2 and 4 samples were 19.0, 7.0 and 11.0%, respectively; their anticoagulant activities were 1/37, 1/62 and 1/205 of heparin, individually. It should be noted that the anticoagulant activity of No.4 sample was decreased to 1/205 of heparin. Thus, this heparin derivative was non-anticoagulant.

Table 2 illustrated the chemical properties, such as uronic acids, hexosamines, free amino groups, reducing powers and average molecular weights for heparin and the No. 4 sample (N-desulfated heparin) as well as their corresponding ratios.

Table 2:

| Measurements of chemical moieties of the sample No. 4. | | | | | |
|---|---|---|---|---|---|
| **Compound** | **Uronic acid (%)** | **Hexosamine (%)** | **TNP-NH$_2$ (Abs 348)** | **Reducing Power (Abs 450)** | **MW** |
| UFH | 26.7 | 23.6 | 0.109 | 0.154 | 19,500 |
| No.4 | 33.0 | 24.9 | 0.838 | 0.179 | 16,100 |
| No.4/UFH | 1.24 | 1.06 | 7.7 | 1.16 | 0.826 |

Table 2: (continued)

| Measurements of chemical moieties of the sample No. 4. | | | | | |
|---|---|---|---|---|---|
| Compound | Uronic acid (%) | Hexosamine (%) | TNP-NH$_2$ (Abs 348) | Reducing Power (Abs 450) | MW |
| UFH: unfractionated heparin; No.4: No.4 N-desulfated heparin sample; Abs: absorbance in 384 nm; MW: average molecular weight (Daltons) | | | | | |

Table 2 showed that the contents of uronic acid and hexosamine in the heparin chain increased 24% and 6%, respectively, after the chemical modification of N-desulfation. As the result of N-desulfation, the more amounts of free amino acid were generated. Compared to heparin, the No. 4 sample had a 7.7-fold increase of the free amino acid content. Further, the chemical modification made the oligosaccharide chain of the No.4 sample shortened 17.4% and the average molecular weight decreased from 19500 Daltons to 16100 Daltons. In contrast, the reducing power increased 16%.

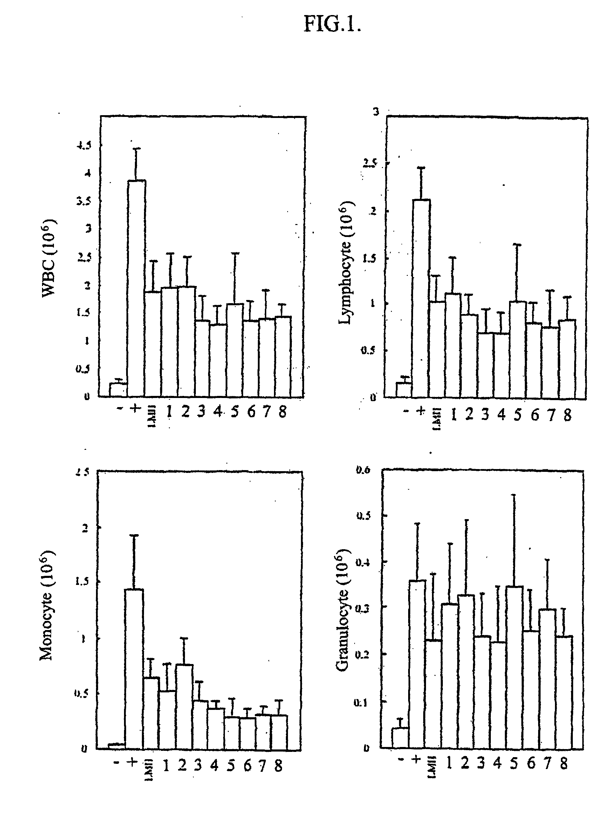

Using the acute peritonitis model induced by the intraperitoneal injection of thioglycollate into mice, LMH and all heparin derivatives were intravenously administered to examine the inhibitory effects of leukocyte peritoneal infiltration including lymphocytes, monocytes and neutrophils. Figure 1 shows that the anti-inflammatory activities of all heparin derivatives were either more potent or equal to that of LMH. Thus, these desulfated heparin derivatives containing 0.1-99.9% N-sulfate of heparin as the starting material can be used to prevent and to treat inflammation diseases.

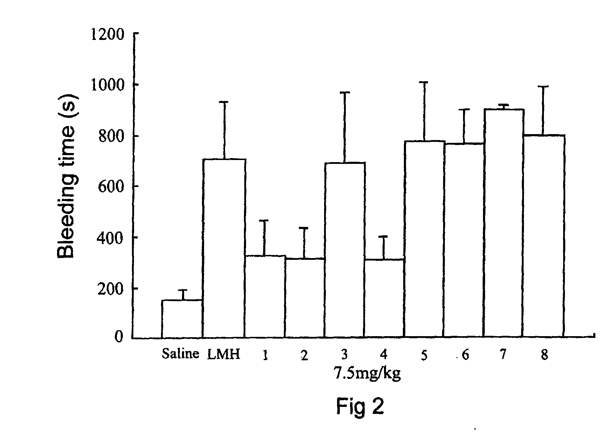

Likewise, LMH and all heparin derivatives (7.5mg/kg) were intravenously administered to test their effects on the bleeding times. Figure 2 shows that the anticoagulant activities in vivo for No.1, 2 and 4 samples were lower than that for LMH, while the anticoagulant activities for the remaining were equal to or a little higher than that for LMH.

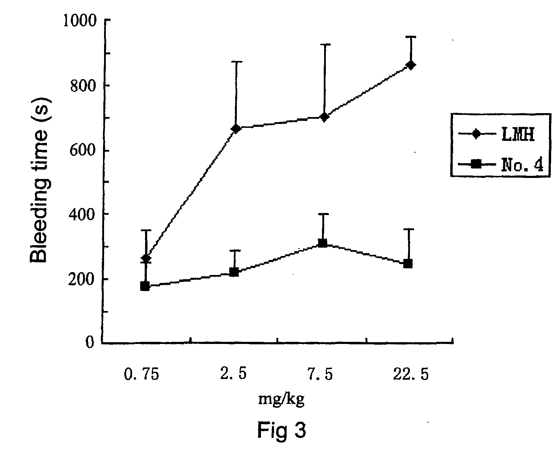

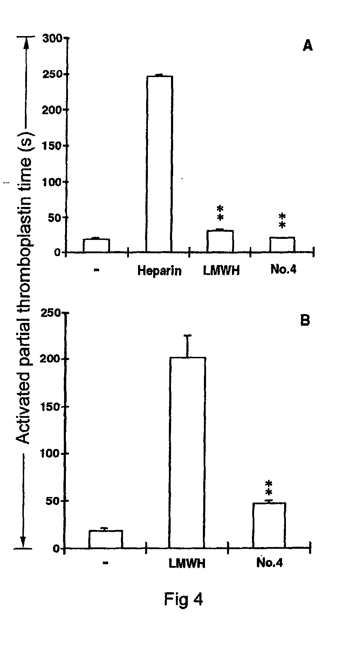

The dose course of bleeding time was carried out to compare the anticoagulant activity of No.4 sample with that of LMH further. Figure 3 demonstrates that following the intravenously injections of 0.75, 2.5, 7.5 and 22.5 mg/kg, the bleeding times for LMH increased sharply while they remained largely unchanged for No. 4 sample. Therefore, No. 4 sample appeared to be non-anticoagulant activity. The conclusion was confirmed by the measurements of APTT in vivo. As shown in Fig.4A, intravenous injection of 2.5 mg/kg of heparin, LMWH and the sample No. 4 could trigger 17-fold, 1.6-fold (**, $P<0.01$ compared with heparin) and 1.1-fold (**, $P<0.01$ compared with heparin) prolongation of APTT respectively. Following the intravenous injections, 7.5 mg/kg LMWH caused 10.7-fold prolongation of APTT while the same amount of the sample No. 4 only induced 2.4-fold (**, $P<0.01$ compared with LMH) prolongation of APTT (Fig. 4B).

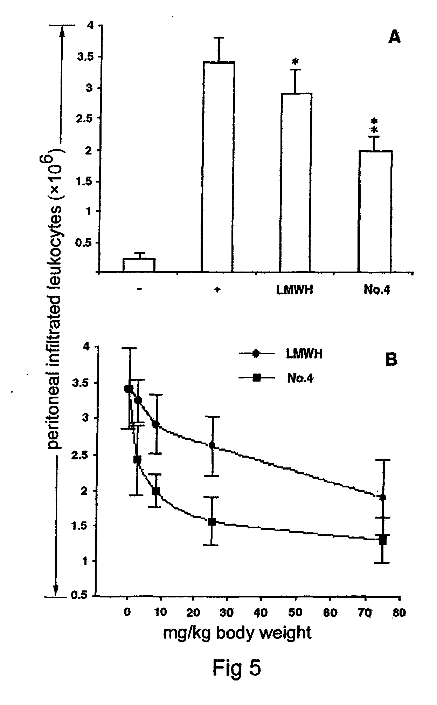

We further compared the anti-inflammatory activities of low molecular weight heparin (LMWH) and the sample No. 4 (N-desulfated heparin) in the mouse model of acute peritonitis using a lower dosage and a dose course. Figure 5A shows that intravenous administration of 10 mg/kg LMWH and the sample No. 4 could significantly reduce the peritoneal deposition of neutrophils with the inhibition of 44.9%. The sample No. 4 appeared to be more potent than LMH (15.7% inhibition). This conclusion was further confirmed by the dose courses of LMWH and the sample No. 4 (Figure 5B). The sample No. 4 was at least 10-fold more potent than LMWH for prevention of neutrophil infiltration in this *in vivo* model.

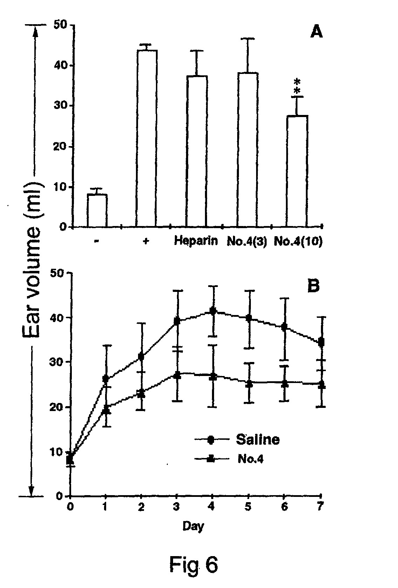

We investigated whether the sample No. 4 (N-desulfated heparin) could reduce ischemia and reperfusion injury using a rabbit ear model. Figure 6A shows that compared to normal ears, complete blockade of blood flow for 6 h followed by spontaneous reperfusion caused significant tissue edema (5.4-fold increase in the ear volume) when they were measured 4 days after the operations. Intravenous injection of 10 mg/kg of the sample No. 4 markedly reduced tissue edema (3.4-fold increase in the ear volume; $p < 0.05$). In contrast, intravenous injections of 3 mg/kg of the sample No. 4 (4.8-fold increase in the ear volumes; $p > 0.05$) and 1 mg/kg of heparin (4.7-fold increase in the ear volumes; $p > 0.05$) had no statistically significant effects on tissue edema. Figure 6B illustrated the time courses of tissue edema in the operation groups treated with saline and the sample No. 4 (10 mg/kg).

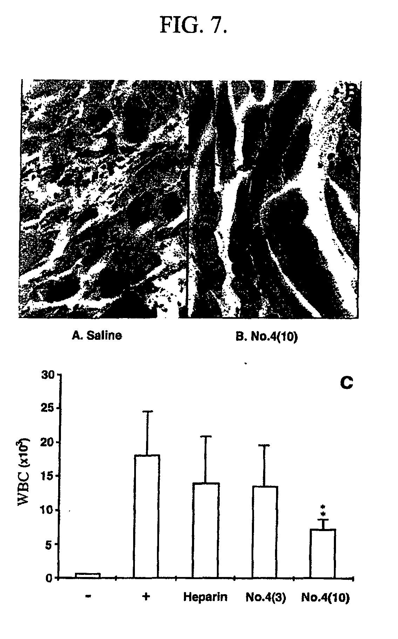

Histological examination and cytologic detection further proved that blocking leukocytes flushing into inflammation sites was one of the anti-inflammation mechanisms for the No.4 sample. Using histological examinations, we found that compared to the tissues from the operation group treated with saline (Figure 7A), 10 mg/kg the sample No. 4 (Figure 7B) significantly decreased the deposition of leukocytes within the injured tissues. We also measured the activities of myeloperoxidease (MPO), an enzyme known to be existed exclusively in neutrophils, to represent the amounts of neutrophils in the injured tissues in each group of animals. Figure 7C shows that compared to the normal tissues, complete blockade of blood flow for 6 h followed by spontaneous reperfusion triggered a substantial amount of neutrophils deposited in the injured tissues. The sample No. 4 (10 mg/kg) significantly inhibited the neutrophil depositions ($p < 0.05$). However, 1 mg/kg heparin ($p > 0.05$) and 3 mg/kg the sample No. 4 ($p > 0.05$) had no statistically significant effects on the neutrophil depositions in the injured tissues after the operations.

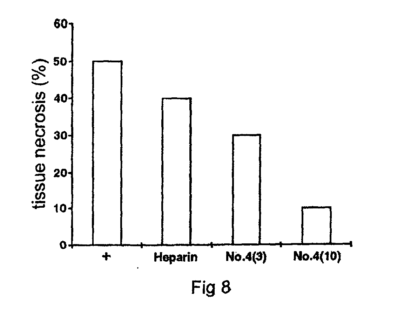

Figure 8 shows that the incidence of tissue necrosis was markedly reduced by treatment with 10 mg/kg the sample No. 4 and less markedly reduced by treatment with 3 mg/kg the sample No. 4. In contrast, the incidence of tissue necrosis was only very moderately reduced after treatment with intravenous administration of heparin (1 mg/kg).

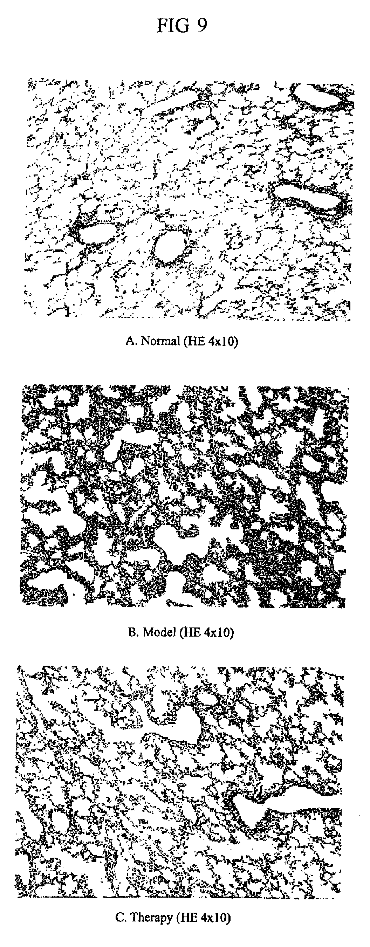

We also investigated the effects of No. 4 sample on acute lung injury using a piglet model. Figure 9 showed the pathological changes in this in vivo model. The histological examinations of the lungs from the normal group indicated the homogeneous dilation of alveoli. There had no apparent edema, hemorrhage, congestion, leukocyte infiltration and other pathological changes (Figure 9A). In sharp contrast, the lungs from the model group showed macroscopically extensive edema, perennial hemorrhage, congestion, and collapse of partial lung tissues. Microscopically, there had massive edema, congestion, hemorrhage and leukocyte infiltration as well as heterogeneous dilation, excessive inflation and collapse of alveoli (Figure 9B). In addition, there had the mild injury of epithelia in the small air passages. However, the lungs from the treatment group of No.4 sample, the edema, congestion, hemorrhage, and leukocyte infiltration were significantly attenuated. There had less exudations in the alveoli, more narrow of the desmohemoblasts among alveoli. The alveoli remained homogeneously dilated, but the mild injury of epithelia in the small air passages were present (Figure 9C).

In addition, a mouse delayed-type hypersensitivity (DTH) model induced by picryl chloride (PC1) was used to evaluate the efficacy of No.4 sample. Table 3 shows that the ALT levels of in the positive control group were markedly increased if compared with those in the normal group. Treatment with 10 mg/kg and 20mg/kg of No. 4 sample markedly reduced the ALT levels (93% and 87% reductions), similar to the group treated with 10 mg/ml cyclophosphamide (87% reduction).

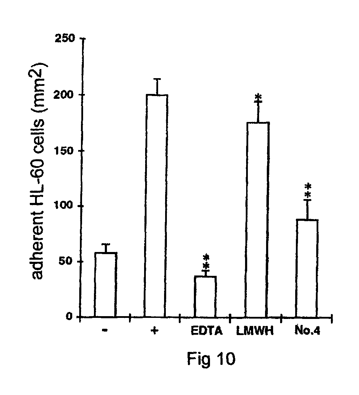

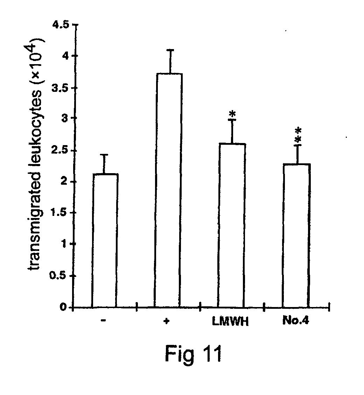

To gain insight into the cellular mechanisms, two in vitro model systems were used to investigate the anti-inflammatory actions of No. 4 sample. Our data clearly demonstrated that No. 4 sample could inhibit the adhesion and transendothelial migration of leukocytes, both of which were essential in inflammatory responses. Figure 10 shows that under a physiological shear stress (2.0 dyne/cm$^2$), adhesion of HL-60 cells to stimulated HUVECs was significantly inhibited by 1 mg/ml the sample No. 4, and the inhibition rate was 78.5% (**, $P<0.01$). But the same concentration of LMWH only mildly neutralized the adhesion of HL-60 cells under flow (17.4%, *, $P<0.05$). We then tested whether these compounds could interfere with the transmigration of human neutrophils through the monolayers of HUVECs following TNF-$\alpha$ stimulation. Figure 11 shows that compared to the unstimulated HUVECs (designated as -), neutrophils migrated more avidly through the monolayers of TNF-$\alpha$ stimulated HUVECs (designated as +). This increased transmigration of neutrophils could be attenuated by 1 mg/ml of both LMWH and the sample No. 4. The inhibition rate of the No.4 sample was 90.6% (**, $P<0.01$) while that of LMH was 70% (*, $P<0.05$). These data clearly demonstrated that No. 4 sample was more potent than LMWH for inhibition of the adhesion and transendothelial migration of leukocytes.

SUMMARY OF THE INVENTION

In one embodiment, the present invention comprises the preparation of a chemically modified heparin. This chemically modified heparin is defined as an N-desulfated heparin, which results in ~89% reduction of the N-sulfate content. This compound has a significantly reduced anticoagulant activity (more than 99% reduction); it will be referred to as N-desulfated heparin thereafter.

In a further embodiment, the invention comprises the use of N-desulfated heparin for prevention and treatment of various acute, subacute and chronic inflammation, conditions characterized by leukocyte infiltration and deposition in the sites of tissue injury leading to further damage of the tissues and organs as well as their consequently functional deteriorations.

BRIEF DESCRIPTION OF THE DRAWING

Table 1 illustrates the relative and absolute amounts of N-sulfate and measurements of activated partial thromboplastin time in heparin, low molecular weight heparin and the chemically modified heparin derivatives.

Figure 1 shows the effects of intravenous injection of small molecular weight heparin and the chemically modified heparin derivatives on the peritoneal infiltration of total leukocytes, lymphocytes, monocytes and neutrophils in a mouse model of acute peritonitis induced by intraperitoneal injection of thioglycollate.

-: Sterile pyrogen-free saline group

+: Positive control group

LMH represents the low molecular weight heparin therapeutic group

1, 2, 3, 4, 5, 6, 7, 8 represent the N-desulfated heparin groups, which contain

19.0, 17.0, 46.2, 11.0, 61.2, 71.0, 86.2 and 64.0% of N-sulfate, respectively.

Figure 2 shows the effects of the chemically modified heparin derivatives on the bleeding times following intravenous injection of mice.

Saline: Sterile pyrogen-free saline group

LMH: low molecular heparin therapeutic group

1, 2, 3, 4, 5, 6, 7, 8 represent the N-desulfated heparin groups, which contain 19.0, 17.0, 46.2, 11.0, 61.2, 71.0, 86.2, 64.0% of N-sulfate, respectively.

Figure 3 shows the dose course of the effects of small molecular weight heparin and N-desulfated heparin (No. 4 sample) on the bleeding times following intravenous injection of mice.

Figure 4 shows the results of aPTT in vivo for heparin, LMWH and No. 4 sample (N-desulfated heparin).

-: Sterile pyrogen-free saline group

Heparin: heparin group

LHM: low molecular weight heparin group

No.4: No. 4 sample group

Figure 5 shows the dose courses for No. 4 sample and low molecular weight heparin in mice peritonitis model (Figure 5B) and the anti-inflammation activity at the dose of 10 mg/kg (Figure 5A).

-: Sterile pyrogen-free saline group

+: Positive control group

LHM: low molecular weight heparin group

No.4: No.4 sample group

Figure 6 shows the effect of No. 4 sample in a rabbit ear model of ischemia and reperfusion injury.

Fig.6A shows the effect on the day 4 after the operation:

-: Normal group

+: Positive control group

Heparin: heparin treatment group at dose of 1mg/kg

No.4(3): No. 4 sample group at 3 mg/kg

No.4(10): No. 4 sample group at 10 mg/kg

Figure 6B shows the time curve of the effects of No. 4 sample:

Saline: Sterile pyrogen-free saline group

No.4: No. 4 sample group

Figure 7 shows the histological examinations for the effects of No.4 sample on the rabbit ear model of ischemia and reperfusion injury.

Figure 7A shows the leukocyte staining within the injured tissues:

Saline: Sterile pyrogen-free saline group

No.4: No. 4 sample group

Figure 7B shows the amounts of neutrophils in the injured tissues:

-: Normal control group

+: Positive control group

Heparin: heparin treatment group at 1 mg/kg

No.4(3): No. 4 sample group at 3 mg/kg

No.4(10): No. 4 sample group at 10 mg/kg

Figure 8 shows the result of that the sample No. 4 markedly reduced the incidence of tissue necrosis.

+: Positive control group

Heparin: heparin treatment group at dose of 1mg/kg

No.4(3): the No.4 sample group at dose of 3mg/kg

No.4(10): the No.4 sample group at dose of 10mg/kg

Figure 9 shows the effect of the No.4 sample in the

A: Normal control group

B: Model control group

C: treatment group of the No.4 sample

Figure 10 shows the inhibitory effects of No.4 sample on the adhesion of leukocytes to endothelial cells:

-: Sterile pyrogen-free saline group
+: Positive control group
EDTA: a chelator for divalent cations
LHM: low molecular weight heparin group
No.4: No. 4 sample group

Figure 11 shows the inhibitory effects of No. 4 sample on the transendothelial migration of leukocytes.

-: Sterile pyrogen-free saline group
+: Positive control group
LHM: low molecular weight heparin group
No.4: No. 4 sample group

DETAILED DESCRIPTION OF THE INVENTION

In accordance with the present invention, a method for treatment of inflammation is provided, which comprises administration of a therapeutically effective amount of N-desulfated heparin. In the method of the present invention, the term "therapeutically effective amount" means the total amount of N-desulfated heparin that is sufficient to show a meaningful patient benefit, that is, the healing of pathological conditions characterized by leukocyte infiltration and deposition or increase in rate of healing of such conditions, whether administered in combination, serially or simultaneously.

In practicing the method of treatment of this invention, a therapeutically effective amount of N-desulfated heparin is administered to a mammal having a disease state. Such disease states include inflammatory disorders such as various kinds of arthritis, asthma, dermatitis and psoriasis, acute respiratory distress syndrome, ulcerative colitis, various types of hepatitis, ischemia/reperfusion injury (including myocardial, renal, skeletal muscular, intestinal, cerebral and pulmonary ischemia/reperfusion injury), shock, severe trauma and transplant rejection.

In practicing the method of the present invention, N-desulfated heparin may be administrated alone or in combination with other therapies. For example, the non-anticoagulant heparin may optionally be used in combination with certain cytokines, lymphokines, or other hematopoietic factors such as M-CSF, GM-CSF, NKSF, IL-1, IL-2, IL-3, IL-4, IL-5, IL-6, IL-7, IL-8, IL-9, IL-10, IL-11, IL-12, G-CSF, Meg-CSF, and erythropoitin to treat inflammatory states. It is contemplated that the method of treatment will allow the non-anticoagulant heparin to synergize with the cytokine, lymphokine, or other hematopoietic factor, thereby augmenting the anti-inflammatory response. Alternatively, the method of treatment will allow N-desulfated heparin to minimize the potential side effects caused by the cytokine, lymphokine, or other hematopoietic factor.

Pharmaceutical compositions used to practice the method of the present invention may contain, in addition to N-desulfated heparin, pharmaceutically acceptable carries, diluents, fillers, salts, buffers, stabilizers, and/or other materials well known in the art. The term "pharmaceutically acceptable" means a non-toxic material that does not interfere with the effectiveness of the biological activity of the active ingredient(s). The characteristics of the carrier or other material will depend on the route of administration.

Administration of N-desulfated heparin used to practice the method of the present invention can be carried out in a variety of conventional ways, such as oral ingestion, or cutaneous, subcutaneous, or intravenous injection. Intravenous administration to the patient is preferred.

When a therapeutically effective amount of N-desulfated heparin is administered orally, the non-anticoagulant heparin will be in the form of a tablet, capsule, powder, solution or elixir. When administrated in tablet form, the pharmaceutical composition of the invention may additionally contain a solid carrier such as a gelatin or an adjuvant. The tablet, capsule, and powder contain from about 5 to 95% N-desulfated heparin, and preferably from about 25 to 90% N-desulfated heparin. When administered in liquid form, a liquid carrier such as water, petroleum, oils and animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of N-desulfated heparin, and preferably from about 1 to 50% N-desulfated heparin.

When a therapeutically effective amount of N-desulfated heparin is administered by intravenous, cutaneous or subcutaneous injection, N-desulfated heparin will be in the form of a pyrogen-free, parentally acceptable solutions, having due regard to pH, isotonicity, stability, and the like, is within the skill in the art. A preferred pharmaceutical composition for intravenous, cutaneous, or subcutaneous injection should contain, in addition to N-desulfated heparin, an isotonic vehicle such as Sodium Chloride Injection, Ringer's Injection, Dextrose Injection, Dextrose and Sodium Chloride Injection, Lactated Ringer's Injection, other vehicle as known in the art. The pharmaceutical composition used to practice the method of the present invention may also contain stabilizers, preservatives, buffers, antioxidants, or other additive known to those of skill in the art.

The amount of N-desulfated heparin in the pharmaceutical composition used to practice the method of the present invention will depend upon the nature and severity of the condition being treated, and on the nature of prior treatments which the patient has undergone. Ultimately, the attending physician will decide the amount of N-desulfated heparin with which to treat each individual patient. It is contemplated that the various pharmaceutical compositions should contain about 0.1 μg to about 100 mg of N-desulfated heparin per kg body weight.

The duration of intravenous therapy using the pharmaceutical composition used to practice the method of the present invention will vary, depending on the severity of the disease being treated and the condition and potential idiosyncratic response of each individual patient. It is contemplated that the duration of each application of N-desulfated heparin will be in the range of 12 to 24 hours of continuous intravenous administration. Ultimately the attending physician will decide on the appropriate duration of intravenous therapy using the pharmaceutical composition of the present invention.

The pharmacokinetics and the toxicity experiments of the N-desulfated heparin are being carried now.

### EXAMPLE 1

### CHEMICAL MODIFICATIONS OF HEPARIN

### Preparation of Pyridine Salt of Heparin

The sodium salt of heparin (Sigma) was passed through a column of Dowex (Strongly Acidic Cation Exchanger 50×4-100; Sigma) and the effluent was neutralized with pyridine. It was then lyophilized to give a white powder [Inoue and Nagasawa, Carbohydr. Res. 46, 87-95 (1976)].

### Preparation of N- and O-desulfated heparins

The pyridine salt of heparin (100 mg dissolved in 0.25 ml of double destilled and deionized $H_2O$) was mixed with 4.75 ml of dimethylsulfoxide (DMSO; Sigma) at 50°C for 2 hour (No. 4 sample), 1 hour (No. 1 sample), or 20°C for 3 hours (No. 3 sample). The sample was diluted with an equal volume of $H_2O$ and the reaction was terminated by adjusting pH to 7.0 with 0.1 N NaOH [Nagasawa and Inoue, Carbohydr. Res. 36, 265-271 (1976); Inoue and Nagasawa, Carbohydr. Res. 46, 87-95 (1976); Tiozzo et al., Thromb. Res. 70, 99-106 (1993)].

The sample No. 1 heparin was further dissolved in 1 N NaOH (4% heparin concentration) at 60°C for 4 hours followed by neutralization to pH 7.0 with 1 N HCl (No. 2 sample). The pyridine salt of heparin was dissolved in 1 N NaOH (4% heparin concentration) followed by treatment at 40°C (No. 5 sample) or at 60°C (No. 6 sample) for 4 hours. Further, the sodium salt of heparin was dissolved in 1 N NaOH (4% heparin concentration) followed by treatment at 40°C (No. 7 sample) or at 60°C (No. 8 sample) for 4 hours. Sample was then neutralized to pH 7.0 with 1 N HCl [Tiozzo et al., Thromb. Res. 70, 99-106 (1993); Lloyd et al., Biochem. Pharmacol. 20, 637-648 (1971)].

### Preparation of Final Heparin Derivatives

After chemical modification, these heparin derivatives were individually passed through a column of Amberlite IRA-400 (Strongly Basic Anion Exchanger; Sigma) to remove the free sulfate ions. The effluent was neutralized to pH 7.0 with 1 N NaOH . It was then desalted by passing through a column of Bio-Gel P-2 (Bio-Rad). Heparins were monitored at the optical densities of 214 nm and 230 nm.

### EXAMPLE 2

### MEASUREMENTS OF ACTIVATED PARTIAL THROMBOPLASTIN TIME

Activated partial thromboplastin time (APTT) was measured using fresh human blood from healthy volunteers. The known amounts of heparin, low molecular weight heparin and the chemically modified heparin derivatives were added prior to the determination of APTT using Silimat™ (bio ĕieux sa) as activator. Six assays were performed for each compound and the anticoagulant activity was expressed as the concentration (μg/ml) that doubles the aPTT time (2-aPTT). The higher concentration was parallel to the lower anticoagulant activity (Table 1). The same assay was carried to determine the aPTT time of the mice in vivo (Figure 4).

EXAMPLE 3

MEASUREMENT OF N-SULFATE AMOUNTS

Solution and reagents: 5% sodium nitrite, 33% acetic acid, 3.8% trichloroacetic acid, barium chloride-gelatin reagent (prepared by dissolving 1 g of gelatin in 100 ml of water, incubated at 60°C to make a complete dissolution, then put it at 4°C overnight. The mixture was filtered after adding 0.5 g of barium chloride and was ready to use after standing for 4 hours at room temperature. This reagent was stored at 4°C and could be used for about one week.

N-sulfates in heparin and various chemically modified heparin derivatives were determined by nitrous acid treatment as described [Inoue and Nagasawa, Anal. Biochem. 71, 46-52 (1976)]. Briefly, 0.5 ml of a sample solution was mixed with 0.5 ml of 5% sodium nitrite and 0.5 ml of 33% acetic acid. After shaking, the mixture was incubated at room temperature for 30 min and 4.5 ml of 3.8% trichloroacetic acid was then added. After shaking again, 1.5 ml of the barium chloride-gelatin reagent was added. Following shaking again immediately, the sample was left standing for 20 min and the turbidity of the sample was measured at 500 nm. The absolute N-sulfate mounts of all N-desulfated heparin sample were calculated with $K_2SO_4$ as control, while the relative N-sulfate mounts of all N-desulfated heparin were calculated using the starting heparin as 100%.

The contents of uronic acid of the No.4 sample and heparin were determined according to the method of Bitter and Muir [Anal. Riochem. 4, 330-334 (1962)]. The hexosamine contents were determined according to the method of Elson and Morgan [Biochem. J. 27, 1824-1828 (1993)]. The contents of free amino group, at a 2 mg/ml concentration, were determined as before [Yoshizawa et al., Biochim. Biophys. Acta., 141, 358-365 (1967)]. Reducing power, at a 2 mg/ml concentration, was measured using the 3, 6-dinitrophthalic acid method [Momose et al., Talanta, 4, 33-37 (1960)]. The average molecular weights were determined using the end group analysis [Hopwood and Robinson, Biochem. J. 135, 631-637 (1973)].

EXAMPLE 4

ANTI-INFLAMMATORY HEPARIN SCREENING ASSAY IN VIVO

Balb/c mice (males, 5 weeks old, 20 ± 1g body weight) were purchased from Shanghai Animal Center of Chinese Academy of Sciences. Negative control group contained 8 mice intraperitoneally injected with 1 ml sterile pyrogen-free saline. Fifteen minutes later, mice were intravenously injected with 0.2 ml sterile pyrogen-free saline alone. The positive control group (12 mice) was intraperitoneally injected with 1 ml 3% thioglycollate broth. Fifteen minutes later, mice were intravenously injected with 0.2ml saline. The mice of the anti-inflammation group (7-11 mice) were intraperitoneally injected with 1 ml 3% thioglycollate broth. Fifteen minutes later, mice were intravenously injected with saline containing 1.5 mg of low molecular weight heparin or any of the chemically modified heparin derivatives. Mice were sacrificed at 2 hours. The peritoneal cavities were lavaged with 8 ml of ice-cold PBS containing 10 U/ml of heparin to prevent clotting. They were centrifuged at 1,500 rpm for 5 min. The total peritoneal leukocytes and their differentiations (lymphocytes, monocytes and granulocytes) were measured using Cell-Dyn1700 (Abbot Laboratories, USA).

Summary:

The mice of heparin therapeutic groups appeared to be more active than the mice of positive control group during the experiments time.

Shortly after the injection of the samples, some amounts of blood was bled from the pinpricks of the mice administrating No.4 sample, concomitantly with the shorter coagulation times than those groups with other heparin samples. Further, when collecting the total peritoneal infiltrated leukocytes, the No. 4 sample group showed no inner bleeding phenomena, but other groups had some extent of bleeding. The experimental findings were consistent with our data that No.4 sample had a significantly reduced anticoagulant activity.

Figure 1 showed the results of the anti-inflammation screening assay in vivo, at the same time, Table 2 represented the inhibition percentages of the peritoneal infiltrated leukocytes.

Table 2:

| The inhibition percentages of leukocytes in the mouse peritonitis model. | | | | | | | | | |
|---|---|---|---|---|---|---|---|---|---|
| | LMH(%) | 1 | 2 | 3 | 4 | 5 | 6 | 7 | 8 |
| WBC | 54.4 | 52.8 | 52.1 | 68.8 | 70.9 | 60.6 | 69.1 | 68.0 | 66.9 |

Table 2: (continued)

| The inhibition percentages of leukocytes in the mouse peritonitis model. | | | | | | | | | |
|---|---|---|---|---|---|---|---|---|---|
| | LMH(%) | 1 | 2 | 3 | 4 | 5 | 6 | 7 | 8 |
| Lymphocytes | 54.9 | 49.5 | 62.0 | 72.1 | 72.1 | 59 | 65.9 | 68.0 | 64 |
| Monocytes | 56.2 | 64.7 | 47.6 | 71.2 | 76.3 | 81.7 | 82 | 79.9 | 80.3 |
| Granulocytes | 40.3 | 16.1 | 11.2 | 36.9 | 40.9 | 4.6 | 33.4 | 19.3 | 10.6 |
| LMH: Low molecular heparin; | | | | | | | | | |
| 1, 2, 3, 4, 5, 6, 7, 8 were the same heparin derivatives as in Table 1. | | | | | | | | | |

The inhibition percentages were calculated using the following formula:

$$1 - \frac{\text{(Cell number of all heparin derivatives - Cell number of the negative control group)}}{\text{(Cell number of the positive control group - Cell number of the negative control group)}}$$

All heparin derivatives had different anti-inflammation potencies. The inhibition percentages for total white blood cells were about 52.1-70.9%, for lymphocytes were about 49.5-72.1%, for monocytes were about 47.6-82%, for granulocytes were about 10.6-40.9%. All heparin derivatives were more potent than LMH and No. 4 sample appeared to be the most potent for inhibition of the peritoneal depositions of leukocytes.

EXAMPLE 5

ANTICOAGULANT ACTIVITY SCREENING ASSAY IN VIVO

Swiss mice (five weeks old, weigh 16g) were purchased from Shanghai Animal Center of Chinese Academy of Sciences. Each group contained 9-11 mice. All the experiments were carried on under 25±1°C, because the bleeding time was varied according to environment temperature. Bleeding times of mice were measured exactly as described previously [Dejana *et al*., Thromb Haemost. 24;48(1):108-11(1982)]. Low molecular weight heparin and chemically modified heparin derivatives (all at 0.12 mg/mouse; 7.5 µg/gram of body weight) were injected into Swiss mice 15 min prior to the tail cutting (2 mm from the tail tip) with a razor blade. For determination of bleeding time, the amputated tail was sunk longitudinally in phosphate buffered saline, pH 7.4, at 25°C. Complete clotting was recorded after stopping the bleeding for 30 sec. And if the bleeding time was longer than 15 min, it was counted as 15 min. Figure 2 shows that following an injection of 120 µg per mouse, No. 1, 2 and 4 samples had significant shorter bleeding times than LMH had, which was just a little longer than those in the saline group.

EXAMPLE 6

DOSE COURSE ASSAY OF ANTICOAGULANT ACTIVITY FOR No. 4 SAMPLE

The assay and the experiment conditions were according to those described in the example 5. Breifly, No. 4 sample and LMH were injected into Swiss mice at 0.75, 2.5, 7.5 and 22.5 mg/kg, respectively. Figure 3 shows that LMH increased the bleeding time sharply while No. 4 sample had no apparent prolongation of the bleeding time. Therefore, No. 4 sample was non-anticoagulant.

EXAMPLE 7

EFFECTS OF No. 4 SAMPLE IN ISCHEMIA AND REPERFUSION INJURY MODEL

Ischemia and reperfusion injury assay. New Zealand *albino* rabbits were purchased from Shanghai Animal Center of Chinese Academy of Sciences. Each group contains 6 rabbits. General anesthesia was induced and maintained by peritoneal injection of pentobarbital sodium (45 mg/kg). After subcutaneous injections of lidocaine at the bases of rabbit ears to block the supplemental local nerves, both ears were carefully amputated at their bases in sterile conditions under a surgical microscope. Only the central artery, the central vein, and a small non-vascular cartilage bridge were left intact. The ear's sensory nerves were transacted to render the ears in a permanently anesthetic condition and the ears were approximated to their bases with suture. A non-traumatic microvascular clip was then placed on the central artery of each ear to stop the blood flow for complete ischemia. After 6 h, the clip was removed and the ear was allowed to spontaneous reperfusion [Mihelcic *et al*., Blood 84, 2322-2328 (1994); Lee *et al*., Surgery 117, 458-465 (1995); Han *et al*., J. Immunol. 155, 4011-4015 (1995)]. For the therapeutic intervention, one bolus of intravenous administration of saline alone, saline with heparin or the No. 4 sample was given at the beginning time of reperfusion (removal of the microvascular clip).

Measurements of tissue edema and necrosis. Ear volume (as a reflection of tissue edema) was measured daily for seven continuous days following removal of the microvascular clip. Ear volume was quantified by determination of the volume displacement after inserting the amputated portion of the ear (to the level of the suture line) into a fluid-filled vessel. Tissue necrosis was assessed, in a double-blind manner, by the presence or absence of tissue necrosis (defined as >5% skin sloughing of the total ear surface) on day 7 [Han *et al., J.Immunol. 155, 4011-4015 (1995)].

Myeloperoxidase (MPO) assay. MPO activities were measured as previously described [Geng *et al*., Nature (Lond.) 343, 757-760 (1990); Schierwagen. *et al*., J. Parmacol. Methods. 23, 179-186 (1990); Mihelcic *et al*., Blood 84, 2322-2328 (1994)]. The ear tissues (no skins) were surgically taken 24 h after reperfusion. They were weighed and placed (0.5 g/ml) in 50 mM potassium phosphate buffer, pH 6.0, supplemented with 0.5 % hexadecyltrimethyl-ammonium bromide (Sigma). They were subsequently homogenized by freeze-thaw three times and sonication twice. The mixtures were then centrifuged at 10,000g for 10 min. The supernatants were heated at 60°C for 2 h to inactivate potential inhibitors of MPO. For generation of standard curves, fresh blood was taken from healthy rabbits and rabbit neutrophils were isolated using Ficoll-Paque Plus (Amersham Pharmacia Biotech) according to the manufacturer's instructions. These isolated neutrophils were more than 95% pure based on differential staining of leukocytes.

Tissue histology. Tissues were taken by the 3-mm punch biopsy from the anesthetic ears 24 h after the operation. Samples were fixed by immersion in Bouin's fixation solution (75% picric acid, 24% formaldehyde, 1% acetic acid) for 24 h at 24°C followed by paraffin embedding. Tissue sections (5-μm thick) were subsequently dewaxed and stained with hematoxylin and eosin. They were photographed at x280.

EXAMPLE 8

EFFECTS OF No. 4 SAMPLE IN ACUTE LUNG INJURY

17 male piglets (6.0-7.4 kg body weight, average 6.7 kg) were purchased from Shanghai Pasturage Institute, Shanghai Academy of Agricultural Sciences (SAAS). The piglets were abrosiaed for 12 hours. Before the operation, the piglets were intramuscularly injected with Prazosin (0.02 mg/kg) and sedated with Ketamine Hydrochloride (7 mg/kg; manufactured by Shanghai Middle West Pharmaceutical Co. Ltd). Fifteen minutes later, the piglets and the scarf skin for the operation and the intubations were cleaned. After establishing the vein bypass, the piglets were given intravenous injection of 2.5 mg/kg Propofol (Fresenius, Germany) to induce anesthesia. They were kept on the infrared constant temperature table (YQT-2; manufactured by Shanghai) on their backs. 0.15 mg/kg Vecuronium Bromide (made by China Xianju Pharmaceutical company, Ltd.) was given intravenously to relax the muscles. A ballonet catheter was inserted through the mouth, which was linked to a respiration machine (Siemens, 900C). After the conventional sterilization and the preparations, the right arteriae femoralis was separated and a 24G cannula was inserted for the collections of the arterial blood samples. Further, it was linked to the equipment for monitoring the hemodynamic data including the artery blood pressures and the heart rates. Thirty minutes later, all data of the basic conditions were started to collected. The piglets were divided into three groups and were operated according to the protocols described below. After the operation, anesthesia was stopped and the piglets became conscious naturally. The tracheal cannulus was withdrawn, when it could breathe independently, $PaO_2$ (partial pressure of oxygen) was lower than 40 mmHg, $SaO_2$ (oxygen saturation) was higher than 95 percent and was reactive to the pain stimulation. The piglet was then kept in the incubator where the temperature was maintained at 21°C. The piglet was intramuscularly injected with Bucinnazine Hydrochloride to ease pain and a glucose-lactate cycle solution (10%, 5 ml/kg/h) was administered intravenously. Twenty-four hours later, the piglet was induced anesthesia by the intravenous injection and intubated into trachea again. The injection speed was adjusted to 10-15 ml/kg/h, according to heart rate, CVP (central venous pressure) and SAP (systemic arterial pressure). After the sterilization, an incision was made in left femoral and a 24G cannulus was inserted for the monitoring of the hemodynamics. An incision was also made in the left venae jugulalis externa and an 18G catheter was inserted for the monitoring of CVP and the collection of venous blood samples. When the conditions of the piglets were stable, the right-lower abdomen was sterilized and sheets laid for the surgery, which varies according to the different groups of piglets, as described below. Gentamycin (20,000 units/piglet) was given intravenously to prevent infection. When acute lung injury appeared, it was treated according to the protocols for each group. Sputum was suctioned every 2 hours during the experiment. In addition, 1 mg/kg Propofol and 0.05 mg/kg Vecuronium Bromide were once injected discontinuously to maintain the breath frequency at 40-50 times per minute and $V_E$ at 0.3 L/min/kg. When acidosis occurred, it was treated with a 5% $NaHCO_3$ solution. When SAP was lower than 60 mmHg, Dopamine was administered intravenously to maintain the blood pressure. At the end of the experiment, the animal was sacrificed with an intravenous injection of 10 ml 10% KCI solution.

According the experimental designs, the piglets were divided randomly into three groups:

Group A (Control Group, n=5): An incision at the right-lower abdomen (3 cm) was made and the intestines were agitated before the incision was closed. Twenty-four hours later, the incision was opened and the intestines were agitated again. The incision was then closured after the peritoneal cavity was washed with 200 ml saline (37°C). The animal was observed for 12 hours.

Group B (Model Group, n=6): An incision at the right-lower abdomen (3 cm) was made followed by a 2-cm perforation in the distal cecum (5 cm from the end). It was sewed and 0.5 cm mucosa was evaginated in order to form an ostium (2 cm in diameter). The incision was then closured surgically. Twenty-four hours later, the incision was opened again and the ostium was sewn up. The incision was eventually closured after washing the peritoneal cavity with 200 ml saline (37°C). The machinery gassing was given until acute lung injury occurred.

Group C (Therapy Group, n=6): All procedures were exactly identical to Group B except that 12 mg/kg No. 4 sample was intravenously administered in early phase of acute lung injury.

Criteria for the determination of acute lung injury:

A. $PaO_2/FiO_2 < 300$ mmHg ($PaO_2$: Partial pressure of oxygen; $FiO_2$: Fraction inspired oxygen concentration)
B. Pulmonary dynamics compliance (Cdyn) lower than that in basic baseline conditions by 30%.

Pathology Observations:

Perfusion fixation of the lungs: Piglets were heparinized. The thorax was opened and the left and right main bronchia were separated. The left lung was inflated with air pressure of 30 $cmH_2O$. One minute later, some air was let out to keep the pressure at about 10 $cmH_2O$. Meanwhile, the right ventricle and the left atria were opened and perfused with 4% formalin at pressure of 65 $cmH_2O$ for 30 minutes.

The left lung was kept in 4% formalin. Three days later, the lung tissue was taken at 0, 2, 6 and 9 points, respectively, in order to minimize the effects of the gravity on the pathological changes. Tissue samples were subsequently dehydrated, embedded with paraffin, sectioned and stained with hematoxylin and eosin (HE).

Tissue sections were examined under a optical microscope and they were classified into 5 grades according to edema, bleeding, leukocyte infiltration, and the pathological changes caused by the epithelial injury of small airways: 0 grade means normal; 1 grade means that the pathological changes are light and the areas of changes are limited; 2 grade means that the pathological changes are moderate and the areas of changes are limited; 3 grade means that the pathological changes are moderate, but they are extensive or obvious in some areas; 4 grade means that the pathological changes are extensive and widespread.

EXAMPLE 9

EFFECTS OF No. 4 SAMPLE ON ACUTE LIVER INJURY

Hypersensitivity was induced in Swiss mice (18-22g body weight) by smearing of 1% (w/v) PCI in absolute ethanol solution on the ventral hair razed abdomen for 5 consecutive days. After another five days, 0.5% PCI olive oil solution (Sigma) was intrahepatically injected. Blood samples were collected 18 hours for the measurements of alanine aminotransferase (ALT). After the intrahepatical injection of PCI, saline, No. 4 sample (10 or 20 mg/kg) or cyclophosphamide (10 mg/kg) were intraperitoneal administered twice at 0 and 5 hours. Table 3 shows that the levels of ALT in the positive control group were markedly increased compared to the normal group. Treatment with No. 4 sample (both dosages) or cyclophosphamide markedly reduced the ALT levels.

Table 3.

| The effects of No. 4 sample on acute liver injury in the PCI-DTH mice | | | |
|---|---|---|---|
| Group | Dosage (mg/kg) | Number of mice | ALT (Karmen unit) |
| Normal | -- | 5 | $21.9 \pm 12.7$ |
| Positive control | -- | 8 | $172.6 \pm 78.2$** |
| No.4 sample | 10 | 10 | $32 \pm 15.9$## |
| No.4 sample | 20 | 9 | $40.3 \pm 36.2$## |
| Cyclophosphamide | 10 | 8 | $40.4 \pm 9.4$## |

\*\*$P<0.01$(compared with normal), ##$P<0.01$(compared with positive control).

EXAMPLE 10

EFFECTS OF No. 4 SAMPLE ON LEUKOCYTE ADHESION

Human umbilical vein endothelial cells (HUVECs; less than three passages) were cultured on one-well chamber slide (Nalge Nunc, Naperville, IL) pre-coated with 1% gelatin as previously described [Geng *et al.*, Nature (Lond.) 343, 757-760 (1990)]. For cytokine stimulation, monolayers of HUVECs were incubated with 300 units/ml of tumor necrosis factor-$\alpha$ (TNF-$\alpha$; Promega, Madison, WI) for 12 h. Slides were mounted in a flow chamber as before [Ma and Geng, J. Immunol. 165, 558-565 (2000)].

Human promyeloid HL-60 cells (CCL 240) were purchased from American Tissue Culture Collection (Rockville, MD). They were cultured in RPMI 1640 medium supplemented with 10% heat inactivated newborn bovine calf serum (BCS), 4 mmol/L L-glutamine, 100 units/ml penicillin and 100 $\mu$g/ml streptomycin at 37°C in the presence of 5% $CO_2$.

After washing once with PBS, HL-60 cells were resuspended at $0.5 \times 10^6$/ml in PBS supplemented with 10 mmol/L HEPES, pH 7.4, and 2 mmol/L $CaCl_2$ in the presence of heparin, LMWH and the sample No. 4 at 22°C for 15 min. They were then precisely injected through the flow chamber at 22°C using a syringe pump. The wall shear stress used was 2.0 dyne/cm$^2$. The numbers of bound cells were quantified from videotape recordings of 10-20 fields of view obtained (3 - 4min after flowing cells through the chamber).

EXAMPLE 11

EFFECTS OF No. 4 SAMPLE ON NEUTROPHIL TRANSMIGRATION

Human umbilical vein endothelial cells (HUVECs; 5 x 10$^4$ cells/well) were seeded in the upper chambers of 24-well Transwell® plates (Costar, Cambridge, MA) pre-coated with 1% gelatin. For cytokine stimulation, monolayers of HUVECs were incubated with 300 units/ml of TNF-$\alpha$ for 12 h [Geng *et al.*, J. Cell Biol. 137, 743-754 (1997)]. Fresh human blood was obtained from healthy volunteers according to the regulations of Chinese Academy of Sciences. Human neutrophils were isolated using Ficoll-Paque Plus (Amersham Pharmacia Biotech, Shanghai, China) according to the manufacturer's instructions. The isolated neutrophils were more than 95% pure based on differential staining of leukocytes. After washing the upper chambers twice, human neutrophils (2.5 x 10$^6$ cells/ml; more than 95% purity) were resuspended in serum-free M199 medium, in the presence of heparin, LMWH and the sample No. 4, and added to the upper chambers of the Transwell plates for 1 h. The upper wells were then removed and the cells sticking on the lower surface of the filters were released by repeated pipetting. Cells in the lower chambers were counted using a hemocytometer.

**Claims**

1. A method of using N-desulfated heparin for prevention and treatment of acute, subacute and chronic inflammation, said the relative N-sulfate content of N-desulfated heparin is 0.1-99.9%.

2. The method of claim 1 wherein said N-desulfated heparin can be administered alone.

3. The method of claim 1 wherein said N-desulfated heparin can be used in combination with certain cytokines, lymphokines, or other hematopoietic factors such as M-CSF, GM-CSF, NKSF, IL-1, IL-2, IL-3, IL-4, IL-5, IL-6, IL-7, IL-8, IL-9, IL-10, IL-11, IL-12, G-CSF, Meg-CSF, and erythropoitin to treat inflammatory states.

4. The method of claim 1 wherein said the pharmaceutical compositions used to practice the method of the present invention may contain, in addition to N-desulfated heparin, pharmaceutically acceptable carries, diluents, fillers, salts, buffers, stabilizers, and/or other materials.

5. The method of claim 1 wherein said N-desulfated heparin can be administered by oral ingestion, or cutaneous, subcutaneous, or intravenous injection. Intravenous administration to the patient is preferred.

6. The method of claim 1 wherein said the intravenous administration of N-desulfated heparin to the patient is preferred.

7. The method of claim 6 wherein said the duration of each application of N-desulfated heparin will be in the range of 12 to 24 hours of continuous intravenous administration.

8. The method of claim 5 wherein said N-desulfated heparin, when orally administered, can be in the form of a tablet, capsule, powder, solution or elixir.

9. The method of claim 8 wherein said when orally administrated the tablet, capsule, and powder contains from about 5 to 95% N-desulfated heparin.

10. The method of claim 8 wherein said when administered in liquid form, the pharmaceutical composition contains from about 0.5 to 90% by weight of N-desulfated heparin.

11. The method of claim 1 wherein said the pharmaceutical composition should contain about 0.1 $\mu$g to about 100 mg of N-desulfated heparin per kg body weight.

FIG.1.

Fig 2

Fig 3

Fig 4

Fig 5

Fig 6

FIG. 7.

A. Saline                    B. No.4(10)

C

Fig 8

## FIG 9

A. Normal (HE 4x10)

B. Model (HE 4x10)

C. Therapy (HE 4x10)

Fig 10

Fig 11

# INTERNATIONAL SEARCH REPORT

International application No.

PCT/CN01/00922

## A. CLASSIFICATION OF SUBJECT MATTER

IPC 7: A61K 31/727

According to International Patent Classification (IPC) or to both national classification and IPC

## B. FIELDS SEARCHED

Minimum documentation searched (classification system followed by classification symbols)

IPC : A61K

Documentation searched other than minimum documentation to the extent that such documents are included in the fields searched

China Pharmaceutical Abstracts

Electronic data base consulted during the international search (name of data base and, where practicable, search terms used)

WPI(Derwent), CNPAT(CN), PAJ(JP), CA

## C. DOCUMENTS CONSIDERED TO BE RELEVANT

| Category* | Citation of document, with indication, where appropriate, of the relevant passages | Relevant to claim No. |
|---|---|---|
| A | EP 0251134 A2 (Hadassa Medical Organization et. al.), 1988.1.7 | 1-11 |
| A | WO 9319734 (Baker Norton Pharmaceuticals, INC.), 1993.10.14 | 1-11 |
| A | EP 0254067 A2 (Hadassa Medical Organization et. al.), 1988.1.27 | 1-11 |
| A | US 5795875 (Kevin R. Holme et. al.), 1998.8.18 | 1-11 |
| A | US 5296471 (Kevin R. Holme et. al.), 1994.3.22 | 1-11 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 2001.09.27 | 2 5 OCT 2001 (2 5. 1 0 0 1) |

| Name and mailing address of the ISA/CN | Authorized officer |
|---|---|
| 6 Xitucheng Rd., Jimen Bridge, Haidian District, 100088 Beijing, China | An Peidong |
| Facsimile No. 86-10-62019451 | Telephone No. 86-10-62093892 |

Form PCT/ISA /210 (second sheet) (July 1998)

## INTERNATIONAL SEARCH REPORT
Information on patent family members

International application No.
PCT/CN01/00922

| Patent document cited in search report | Publication date | Patent family member(s) | Publication date |
|---|---|---|---|
| EP 0251134 A2 | 1988.01.07 | AU7470687 A | 1988.01.07 |
|  |  | DK8703312 A | 1987.12.27 |
|  |  | JP63088127 A | 1988.04.19 |
|  |  | DE3782686 | 1992.12.24 |
|  |  | US5206223 | 1993.04.27 |
|  |  | CA1318591 | 1993.06.01 |
|  |  | ES2052514 | 1994.07.16 |
| WO 9319734 | 1993.10.14 | AU3938093 | 1993.11.08 |
| EP 0254067 A2 | 1988.01.27 | NO8702674 A | 1988.01.18 |
|  |  | AU7470587 A | 1988.01.07 |
|  |  | DK8703313 A | 1987.12.27 |
|  |  | JP63088128 A | 1988.04.19 |
|  |  | US4882318 A | 1989.11.21 |
|  |  | CA1318591 | 1993.06.01 |
| US 5795875 | 1998.8.18 | WO 9530424 A1 | 1995.11.16 |
|  |  | AU2435795 A | 1995.11.29 |
|  |  | EP0758247 A1 | 1997.02.19 |
|  |  | JP9512822 | 1997.12.22 |
| US 5296471 | 1994.3.22 | WO9414851 A1 | 1994.07.07 |
|  |  | AU5404094 A | 1994.07.19 |
|  |  | EP0862585 A1 | 1998.09.09 |

Form PCT/ISA /210 (patent family annex) (July 1998)

## INTERNATIONAL SEARCH REPORT

International application No.

PCT/CN01/00922

**Box I**     **Observations where certain claims were found unsearch able (Continuation of item 1 of first sheet)**

This international search report has not been established in respect of certain claims under Article 17(2)(a) for the following reasons:

1. ☐   Claims Nos:
    because they relate to subject matter not required to be searched by this Authority, namely:

2. ☐   Claims Nos:
    because they relate to parts of the international application that do not comply with the prescribed requirements to such an extent that no meaningful international search can be carried out, specifically:

3. ☐   Claims Nos:
    because they are dependent claims and are not drafted in accordance with the second and third sentences of Rule 6.4(a)

**Box II**     **Observations where unity of invention is lacking (Continuation of item 2 of first sheet)**

This International Searching Authority found multiple inventions in this international application, as follows:

1. ☐   As all required additional search fees were timely paid by the applicant, this international search report covers all searchable claims.

2. ☐   As all searchable claims could be searched without effort justifying an additional fee, this Authority did not invite payment of any additional fee.

3. ☐   As only some of the required additional search fees were timely paid by the applicant,this international search report covers only those claims for which fees were paid, specifically claims Nos.:

4. ☐   No required additional search fees were timely paid by the applicant.Consequently ,this international search report is restricted to the invention first mentioned in the claims;it is covered by claims Nos.:

**Remark on protest**      ☐   The additional search fees were accompanied by the applicant's protest.

            ☐   No protest accompanied the payment of additional search fees.

Form PCT/ISA /210 (cotinuation of first sheet (1)) (July 1998)